Europäisches Patentamt

European Patent Office

Office européen des brevets

(11) Veröffentlichungsnummer: **0 021 162**
**B1**

(12) **EUROPÄISCHE PATENTSCHRIFT**

(45) Veröffentlichungstag der Patentschrift:
**12.05.82**

(21) Anmeldenummer: 80103112.1

(22) Anmeldetag: **04.06.80**

(51) Int. Cl.³: **C 07 C 85/12,** C 07 C 85/24,
C 07 C 87/123

(54) Verfahren zur Herstellung gesättigter sekundärer Alkylamine.

(30) Priorität: **12.06.79 DE 2923686**

(43) Veröffentlichungstag der Anmeldung:
**07.01.81 Patentblatt 81/1**

(45) Bekanntmachung des Hinweises auf die Patenterteilung:
**12.05.82 Patentblatt 82/19**

(84) Benannte Vertragsstaaten:
**BE DE FR GB**

(56) Entgegenhaltungen:
**GB-A-759 291**
**GB-A-836 364**
**US-A-3 673 251**

(73) Patentinhaber: **HOECHST AKTIENGESELLSCHAFT,**
**Postfach 80 03 20, D-6230 Frankfurt/Main 80 (DE)**

(72) Erfinder: **Müller, Heinz, Dr., Kantsstrasse 43,**
**D-8261 Burgkirchen/Alz (DE)**
Erfinder: **Becker, Adolf, Gerhard-Hauptmann-Strasse 6,**
**D-8261 Burgkirchen/Alz (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

## Verfahren zur Herstellung gesättigter sekundärer Alkylamine

Die Erfindung betrifft ein Verfahren zur Herstellung gesättiger sekundärer Alkylamine mit 12 bis 22 Kohlenstoff-Atomen in jeder Alkylkette, wobei diese gleich oder verschieden sein können, durch Hydrierung von ungesättigten Fettsäurenitrilen der entsprechenden Kettenlängen mit 1 bis 3 olefinischen Doppelbindungen im Molekül oder deren Gemischen mit bis zu 70% gesättigten Fettsäurenitrilen der genannten Kettenlängen in Gegenwart von Nickel- oder Kobaltkatalysatoren, wobei der Wasserstoff im wesentlichen im Kreis geführt und Ammoniak aus dem Kreisgas entfernt wird.

Die Herstellung von sekundären Alkylaminen durch Hydrierung von gesättigten und ungesättigten Fettsäurenitrilen ist bekannt.

Insbesondere erhält man nach einem Verfahren, wie es in der GB-PS 836 364 beschrieben ist, sekundäre Amine, wenn man gesättigte oder ungesättigte Fettsäurenitrile und/oder primäre Amine bei Drücken von 1 bis 18,5 bar und Temperaturen von 150 bis 250° C einer katalytischen Hydrierung unterwirft. Hierbei können alle üblichen Hydrierungskatalysatoren verwendet werden, bevorzugt ist der Einsatz von Raney-Nikkel. Es handelt sich dabei um ein diskontinuierliches Verfahren, bei dem der zur Hydrierung verwendete Wasserstoff und der bei der Reaktion gebildete Ammoniak in einer Menge von 1 bis 4 Mol pro Stunde und pro Mol des eingesetzten Ausgangsmaterials kontinuierlich aus dem Reaktionsgefäß abgezogen werden und der Wasserstoff nach dem Auswaschen des Ammoniaks mit Wasser im geschlossenen Kreislauf wieder in die Reaktionszone zurückgeführt wird. Die angewendeten Drücke sollen dabei zwischen 1 und 18,5 bar liegen. Beim Einsatz von Fettsäurenitrilen als Ausgangsmaterialien wird empfohlen, anfangs vorzugsweise höhere Drücke anzuwenden, bis sich aus den Nitrilen Imine und/oder Amine gebildet haben, und erst danach den Druck soweit als möglich in die Nähe des Normaldrucks zu reduzieren.

Da festgestellt wurde, daß die Anwesenheit von Wasser im Reaktionsgemisch sich nachteilig auf die Reaktion auswirkt, wird vorgeschrieben, dieses Wasser, das in dem eingesetzten Nitril oder Amin enthalten ist oder mit dem Katalysator eingebracht wird, vor dem Beginn der Reaktion durch Erhitzen auf 120 bis 130° C unter Rühren und Durchleiten von Stickstoff oder Wasserstoff zu entfernen. Nach den Angaben in der GB-PS 836 364 ist die Reaktion in etwa 2 bis 6 Stunden beendet. Es finden sich jedoch keine Angaben darüber, welche Ausbeuten an sekundären Aminen erzielt werden und inwieweit gesättigte sekundäre Amine aus ungesättigten Nitrilen oder Gemischen von ungesättigten mit gesättigten Nitrilen erhalten werden können.

Während in dem genannten Verfahren zwar der schädliche Einfluß von Wasser in der Reaktionsmischung erkannt wurde, wurde jedoch der nach der Ammoniakwäsche vom Kreisgas tensionsmäßig mitgenommene Wasserdampf-Partialdruck offenbar in Kauf genommen. Wie aus der dort beigefügten Zeichnung hervorgeht, wird das Kreisgas nach dem Passieren des Wäschers unmittelbar in die Reaktionszone zurückgeführt, womit es zwangsläufig mit Wasserdampf gesättigt ist. Diese Wasserdampf-Tension stört zwar bei der Nitrilhydrierung nur wenig, sie verzögert und erschwert aber in erheblichem Maße die Hydrierung olefinischer Doppelbindungen, so daß es auf diese Weise bei Einsatz ungesättigter Nitrile nicht möglich ist, vollständig oder annähernd gesättigte sekundäre Amine in wirtschaftlich sinnvollen Reaktionszeiten herzustellen.

Dieser Nachteil kann gemäß der vorliegenden Erfindung vermieden werden durch das im Oberbegriff des Patentanspruchs 1 genannte Verfahren, das dadurch gekennzeichnet ist, daß unter praktisch drucklosen Bedingungen während der gesamten Reaktionsdauer im Kreisgas vor Rückführung in die Reaktionszone ein Wassergehalt von höchstens 5 g/m$^3$ bei 25° C und Normaldruck eingestellt und/oder aufrechterhalten wird.

Als Ausgangsmaterialien für das erfindungsgemäße Verfahren werden ungesättigte Fettsäurenitrile mit 12 bis 22, vorzugsweise 16 bis 18, Kohlenstoffatomen und mit 1 bis 3 (konjugierten oder vorzugsweise isolierten) Doppelbindungen in der Kohlenstoffkette oder deren Gemische verwendet, die nach bekannten Verfahren aus den entsprechenden Fettsäuren hergestellt werden können. Ebenso geeignet sind auch Gemische aus ungesättigten und gesättigten Nitrilen, die aus den Fettsäuren natürlich vorkommender Fette und Öle hergestellt werden können, wie beispielsweise aus Tranfettsäure und Rübölsäure und insbesondere aus Sojaölsäure und aus Talgfettsäure. Hierin können die ungesättigten Fettsäurenitrile im Gemisch mit 1 bis 70 Gew.-%, vorzugsweise 1 bis 60 Gew.-%, an gesättigten Fettsäurenitrilen mit 12 bis 22, vorzugsweise 14 bis 18, Kohlenstoffatomen vorliegen (das heißt, das Verfahren ist anwendbar auf ungesättigte Fettsäurenitrile mit 12 bis 22, vorzugsweise 16 bis 18 Kohlenstoffatomen, die 1 bis 3 Doppelbindungen in der Kohlenstoffkette enthalten können, und die 0 bis 70, vorzugsweise 0 bis 60 Gew.-%, an gesättigten Fettsäurenitrilen mit 12 bis 22, vorzugsweise 14 bis 18 Kohlenstoffatomen enthalten). Ein besonders bevorzugtes Ausgangsmaterial ist Talgfettnitril, das aus Talg verschiedenen tierischen Ursprungs gewonnen wird, und das aus etwa 40 bis 75 Gew.-%, vorzugsweise 45 bis 60 Gew.-%, an ungesättigtem C$_{16}$-Nitril mit einer Doppelbindung und C$_{18}$-Nitrilen mit 1 bis 3 Doppelbindungen in der Kohlenstoffkette und aus 25 bis 60 Gew.-%, vorzugsweise 40 bis 55 Gew.-%, gesättigten C$_{14}$-, C$_{16}$- und C$_{18}$-Nitrilen zusammengesetzt ist. Ein weiteres besonders bevorzugtes Ausgangsmaterial ist Sojanitril, das aus etwa 70 bis 92 Gew.-%, vorzugsweise 75 bis 90 Gew.-%, an ungesättigten $C_{18}$-Nitrilen mit 1 bis 3 Doppelbindungen in der Kohlenstoffkette und aus 8 bis 30 Gew.-%, vorzugsweise 10 bis 15 Gew.-%, gesättigten $C_{14}$, $C_{16}$- und $C_{18}$-Nitrilen zusammengesetzt ist.

Derartige aus natürlichen Fetten stammende Nitrilgemische können in sehr kleinen Anteilen zusätzlich die höheren und niedrigeren, geradzahligen Homologe enthalten. Die genannten Zahlen an Kohlenstoffatomen schließen den Kohlenstoff der Nitrilgruppe ein.

Zur Durchführung des erfindungsgemäßen Verfahrens gibt man das oder die ungesättigten Fettsäurenitrile, gegebenenfalls im Gemisch mit gesättigten Nitrilen, wie vorstehend beschrieben, in einen Reaktionskessel, der mit einem Heiz- bzw. Kühlmantel und einem gut wirksamen Rührer ausgestattet ist. Außerdem hat der Reaktionskessel Vorrichtungen zum Ein- und Ableiten von Gasen, zum Befüllen und Entleeren sowie zur Kontrolle und Regelung von Druck und Temperatur.

Ferner gibt man in den Reaktionskessel, der Teil einer Kreisgasapparatur ist, den zur Hydrierung des Nitrils vorgesehenen Katalysator. Als Katalysatoren eignen sich Nickel- und Kobaltkatalysatoren, die mit Spuren anderer Metalle wie beispielsweise Calcium, Eisen und Molybdän dotiert sein können und die in Form von Pulverkontakten oder Raney-Katalysatoren eingesetzt werden können. Bei Pulverkontakten eignen sich als Trägermaterial beispielsweise Aluminiumoxid, Silikagel, Kieselgur und Bimsstein. Bevorzugt werden Nickel-Katalysatoren verwendet, insbesondere in Form von Raney-Nickel. Die genannten Katalysatoren werden üblicherweise dem Ausgangsgemisch in Mengen von 0,3 bis 10 Gew.-%, vorzugsweise von 2 bis 5 Gew.-%, zugesetzt.

Falls das Ausgangsgemisch nicht genügend wasserfrei ist, wird es vor Beginn der eigentlichen Hydrierung auf Temperatur über 100°C, vorzugsweise auf Temperaturen von 120 bis 130°C, aufgeheizt und in bekannter Weise, beispielsweise durch Hindurchleiten von Stickstoff oder Wasserstoff unter intensivem Rühren von dem in den Ausgangsmaterialien und/oder dem Katalysator enthaltenem Wasser befreit. Nach Spülen mit Wasserstoff und Aufheizen auf die Reaktionstemperatur, die je nach Art des verwendeten Katalysators und der Natur des eingesetzten Fettsäurenitrils oder Nitrilgemisches im Temperaturbereich von etwa 150 bis 220°C, vorzugsweise im Temperaturbereich von etwa 170 bis etwa 200°C, liegt, wird unter wirksamer Durchmischung Wasserstoff mit der Reaktionsmischung in innigen Kontakt gebracht. Der überschüssige Wasserstoff wird zusammen mit dem bei der Reaktion gebildeten Ammoniak aus dem Reaktionskessel abgezogen, gegebenenfalls in einem Kühler auf 20 bis 50°C abgekühlt, und sodann wird der Ammoniak vollständig oder teilweise aus dem Kreisgas entfernt. Bevor das Kreisgas nun in die Reaktionszone zurückgeführt wird, ist durch die im folgenden beschriebenen Maßnahmen dafür Sorge zu tragen, daß im Kreisgas ein Wassergehalt von höchstens 5 g/m³, vorzugsweise von höchstens 1 g/m³, und insbesondere von höchstens 0,8 g/m³ bei 25°C und Normaldruck, eingestellt und/oder aufrechterhalten wird. Der bei der Reaktion verbrauchte Wasserstoff wird durch Zudosierung von Frischwasserstoff ersetzt. Zur Erzielung einer ausreichenden Hydriergeschwindigkeit ist es zweckmäßig, Wasserstoff im Überschuß anzuwenden, das heißt, etwa 2 bis etwa 10 Mol Wasserstoff pro Mol Fettsäurenitril und pro Stunde im Kreislauf zu führen, wobei die Begrenzung dieser Menge nach oben ausschließlich von wirtschaftlichen Gesichtspunkten abhängt. Bei der Zuführung von Wasserstoff zu Beginn und während der Reaktion ist selbstverständlich darauf zu achten, daß der zugeführte oder nachgeführte Wasserstoff den oben angegebenen Wassergehalt nicht überschreitet.

Die Entfernung des Ammoniaks aus dem Kreisgas geschieht beispielsweise in einem Waschturm, in den das ammoniakhaltige Kreisgas von unten eingeführt und nach oben abgezogen wird. Im Gegenstrom fließt die Waschflüssigkeit von oben nach unten. Zur besseren Durchmischung kann der Wäscher mit Füllkörpern, beispielsweise mit Raschigringen gefüllt oder mit speziellen Einbauten versehen sein.

Als Waschflüssigkeit kommt in erster Linie Wasser in Frage. Verwendet man zur vollständigen oder teilweisen Entfernung des Ammoniaks aus dem Kreisgas Wasser als Waschflüssigkeit, so ist das Kreisgas nach Verlassen des Wäschers tensionsmäßig mit Wasserdampf gesättigt, das sind bei 25°C und Normaldruck immerhin 22,2 g Wasser/m³. Erfindungsgemäß muß daher das Kreisgas nach Verlassen des Wäschers und vor Wiedereintritt in die Reaktionszone bis auf einen Wassergehalt von höchstens 5 g/m³, vorzugsweise von höchstens 1 g/m³ und insbesondere von höchstens 0,8 g/m³, bei 25°C und Normaldruck getrocknet und damit eingestellt werden. Hierfür können übliche feste oder flüssige Trockenmittel eingesetzt werden.

Als feste Trockenmittel sind beispielsweise geeignet Ätznatron, Calciumsulfat und Silikagel. Bei Verwendung von festen Trockenmitteln sind 1 bis 2, zweckmäßigerweise aber 2 Trockentürme vorzusehen, wobei im letzteren Falle sich jeweils der eine im Einsatz befindet, während der andere regeneriert wird.

Als flüssige Trockenmittel können beispielsweise verwendet werden Diethylenglykol, Triethylenglykol, konzentrierte Schwefelsäure oder Orthophosphorsäure. Die Anwendung dieser flüssigen Trockenmittel erfolgt in einer ähnlichen Vorrichtung, wie sie oben für die Wäsche des Ammoniaks beschrieben wurde.

Für die Entfernung des Ammoniaks können aber auch andere Flüssigkeiten eingesetzt werden, die soweit wasserfrei sind, daß sie den Wassergehalt des Kreisgases nicht erhöhen, wodurch dieser im Kreisgas also aufrechterhalten wird. Beispielsweise geeignet sind Diethylenglykol oder Triethylenglykol. Ferner bieten sich zur Entfernung des Ammoniaks auch ungesättigte oder partiell ungesättigte Fettsäuren an, wie sie zur Herstellung der bei dem erfindungsgemäßen Verfahren als Ausgangsprodukte eingesetzten Nitrile benötigt werden. Die sich hierbei bildenden fettsauren Ammoniumsalze bleiben in überschüssiger Fettsäure gelöst. Diese Lösung ist kein Abfallprodukt, sondern sie kann anschließend zur Herstellung von Ausgangsnitrilen unter weiterem Ammoniakzusatz verwendet werden.

Das erfindungsgemäße Verfahren wird unter praktisch drucklosen Bedingungen durchgeführt. Unter praktisch drucklosen Bedingungen soll hier ein Druck von 1 bis etwa 3 bar verstanden werden, wie dies in der Technik üblich ist, da man das Eindringen von Luft in den Reaktionskessel verhindern will.

Zunächst wird im Rahmen des erfindungsgemäßen Verfahrens unter dem Begriff Entfernung von Ammoniak aus dem Kreisgas die praktisch vollständige Ammoniakentfernung verstanden. Bei der Herstellung von gesättigten sekundären Alkylaminen aus ungesättigten Nitrilen oder Gemischen von ungesättigten mit gesättigten Nitrilen muß außer der Hydrierung der Nitrilgruppe und der Bildung des sekundären Amins unter Ammoniakabspaltung auch die Hydrierung der olefinischen Doppelbindung oder Doppelbindungen in der Alkylkette erfolgen. Diese verläuft meist langsamer als die erstgenannten Reaktionen, so daß die gesamte Reaktionsdauer länger ist, als zur Bildung des sekundären Amins erforderlich ist. Mit speziell aktivierten Katalysatoren kann man die Dauer dieser Nachreaktion verkürzen, erhält dann jedoch meist einen etwas höheren Gehalt an tertiären Aminen im Endprodukt, was unerwünscht ist. Es kann daher zweckmäßig sein, das Ammoniak während der Reaktion vollständig aus dem Kreisgas zu entfernen, sondern einen Ammoniakgehalt von 3 bis 50 Vol.-%, vorzugsweise von 3 bis 30 Vol.-%, insbesondere von 5 bis 20 Vol.-%, bezogen auf das Gesamtvolumen des Kreisgases, zuzulassen. Mit anderen Worten kann der Ammoniakgehalt im Kreisgas durch die Ammoniakentfernung auf 0 bis 50, vorzugsweise auf 0 bis 30 Vol.-%, insbesondere auf 0 bis 20 Vol.-%, eingestellt werden. Die Entfernung einer Teilmenge des Ammoniaks kann beispielsweise dadurch bewerkstelligt werden, daß über den obengenannten Ammoniakwäscher nur ein Teilstrom des Kreisgases geleitet wird, dessen regulierbare Menge das Ausmaß der Ammoniakentfernung einstellt, oder aber dadurch, daß man die Waschwassermenge im Ammoniakwäscher verringert. Es wurde nun festgestellt, daß ein feuchtes Kreisgas, das aus Wasserstoff und Ammoniak besteht und einen Wassergehalt über der oben genannten kritischen Grenze aufweist, die Hydrierung der Doppelbindung oder der Doppelbindungen noch stärker behindert als feuchter Wasserstoff allein. Daher hat hier die erfindungsgemäße Einstellung und/oder Aufrechterhaltung eines Wassergehaltes im Kreisgas von höchstens 5 g/m³, vorzugsweise 1 g/m³ und insbesondere von 0,8 g/m², bei 25°C und Normaldruck noch größere Bedeutung als im Falle der Verwendung eines ammoniakfreien Wasserstoffs als Kreisgas.

Die Entfernung oder die Regulierung des Ammoniakgehaltes im Kreisgas kann auch durch Auskondensieren bei entsprechender Abkühlung erfolgen, wobei erfindungsgemäß der Wassergehalt in den gekannten Grenzen aufrechterhalten wird. Ebenso kann auch die erfindungsgemäße Einstellung des Wassergehaltes im Falle der Ammoniakwäsche mit Hilfe von Wasser durch Ausfrieren mittels eines Kühlaggregats erfolgen.

Es wurde nun weiterhin festgestellt, daß sich je nach Temperaturbedingungen und eingesetztem Katalysator (wie beispielsweise Raney-Nikkel) während der Zeitdauer des erfindungsgemäßen Verfahrens im Kreisgas etwas Methan als unerwünschtes Nebenprodukt bilden kann, das sich im Laufe der Reaktionszeit im Kreisgas anreichert, wenn in einem vollständig geschlossenen Kreislauf gearbeitet wird. Bei gleicher Kreisgasmenge sinkt so der Wasserstoff-Partialdruck im Kreisgas ab, wodurch die Reaktionsdauer verlängert wird. Es ist daher von Vorteil, einen Teil des aus dem Reaktionsgefäß abgezogenen Gasgemisches als Abgas zu entfernen. Dieser Anteil kann 1 bis 20 Vol.-%, vorzugsweise 1 bis 10 Vol.-%, des umlaufenden Kreisgases betragen. Eine solche Fahrweise bringt gleichzeitig den Vorteil mit sich, daß mit der Ausschleusung der genannten Teilmenge an Kreisgas aus dem Kreislauf auch Ammoniak entfernt wird, so daß diejenige Menge Ammoniak, die durch die obengenannten anderen Maßnahmen entfernt werden muß, sich entsprechend verringert. Üblicherweise wird die Ausschleusung der genannten Teilmenge des Kreisgases während der gesamten Reaktionszeit kontinuierlich vorgenommen. Es ist jedoch auch möglich, Abgas portionsweise zu entnehmen, wobei die Menge dieser Portionen dann so bemessen ist, daß sie der oben angegebenen, kontinuierlich abgeführten Menge entspricht.

Die nach dem erfindungsgemäßen Verfahren hergestellten gesättigten sekundären Alkylamine werden als Ausgangsmaterialien zur Herstellung von Textilhilfsmitteln Desinfektionsmitteln, Antistatika und organischen Ammoniumbentoniten eingesetzt. Sie sind von heller Farbe und behalten diese helle Farbe auch bei der Quaternisierung zum quaternären Ammoniumsalz bei.

Das erfindungsgemäße Verfahren wird in den folgenden Beispielen näher erläutert:

In allen Beispielen und Vergleichsbeispielen (soweit nicht anders vermerkt) werden 200 g eines Talgfettnitrils eingesetzt, welches einen Stickstoffgehalt von 5,47 Gew.-% und eine Iodzahl von 52,4 aufweist und aus 49,4 Gew.-% an ungesättigten $C_{16}$- und $C_{18}$-Nitrilen (wobei die letzteren 1 bis 3 Doppelbindungen in der Kette aufweisen) und aus 50,6% gesättigten $C_{14}$-, $C_{16}$- und $C_{18}$-Nitrilen zusammengesetzt ist. Als Katalysator werden 5 Gew.-% Raney-Nickel, bezogen auf eingesetztes Nitril, verwendet (soweit nicht anders vermerkt).

Die für die Reaktion verwendete Laborapparatur besteht aus einem 500-ml-Rundkolben, versehen mit Rührer, Kontaktthermometer, einer Gaseinleitungsvorrichtung und einer Gasableitungsvorrichtung, wobei die letztere aus einer auf 90°C beheizbaren Füllkörper-Kolonne mit einem kleinen Abscheider und einem bei 20°C betriebenen aufgesetzten Rückflußkühler besteht. Über diesen Abscheider wird insbesondere vor Beginn der Hydrierung das im Ausgangsgemisch und im Katalysator enthaltene Wasser durch Aufheizen auf 120°C bis 130°C unter Hindurchleiten von Wasserstoff entfernt. Soweit nicht anders vermerkt, wird die Entfernung des Wassers wie folgt vorgenommen: Hinter dem Rückflußkühler befindet sich ein mit Glasfüllkörpern gefüllter Waschturm, in den von unten das Gas eingeleitet und der von oben mit Wasser berieselt wird, wodurch das bei der Hydrierung gebildete Ammoniak aus dem Gasstrom herausgewaschen wird. Anschließend passiert das Gas in den erfindungsgemäßen Beispielen einen Trockenturm mit Ätznatron. Das trockene Gas wird dann mit einer Schlauchpumpe wieder in den Reaktionskolben zurückgeführt. Unmittelbar vor Eintritt des Kreisgases in den Reaktionskolben wird der für die Hydrierung benötigte Wasserstoff im Ausmaß des Verbrauchs dem Kreislauf zugespeist; dieser Wasserstoff besitzt einen Wassergehalt von $< 0,2$ g/m³ bei 25°C und Normaldruck. Mit Hilfe einer Nebenleitung ist es möglich, entweder den Gesamtstrom oder auch nur einen Teilstrom des Kreisgases über Wasch- und gegebenenfalls Trockenturm zu leiten.

Die Reaktionstemperatur beträgt 180°C, die Reaktionszeit in allen Fällen 6 Stunden, die Kreisgasmenge 500 l/kg · h (soweit nicht anders vermerkt). Nach Beendigung der Reaktion wird auf 80°C abgekühlt, der Katalysator abgetrennt und das erhaltene Produkt analysiert.

### Beispiel 1

Wie vorstehend beschrieben, wird das Kreisgas während der Reaktionszeit in seiner Gesamtmenge über den mit Wasser berieselten Ammoniak-Waschturm und anschließend über einen Trockenturm, gefüllt mit Ätznatron, geleitet. Es hat nach dieser Trocknung einen Wassergehalt von 0,7 g/m³ bei 25°C und Normaldruck.

Das erhaltene Amin besitzt eine Aminzahl von 19,17, einen Gehalt an sekundärem Amin von 97,1 Mol-%, an tertiärem Amin von 2,8 Mol-% und an primärem Amin von 0,1 Mol-%. Die Ausbeute an Gesamtamin beträgt 97,6 Gew.-%, bezogen auf eingesetztes Ausgangsnitril. Das Gesamtamin weist eine Iodzahl von 0,7 auf, das heißt, es sind 98,7% der vorhandenen Doppelbindungen hydriert worden.

### Vergleichsbeispiel 1a)

Es wird verfahren wie in Beispiel 1, jedoch wird das Kreisgas nach Durchgang durch den mit Wasser berieselten Ammoniak-Waschturm nicht getrocknet, und es besitzt nach Passieren des Waschturmes einen Wassergehalt von 17,5 g/m³ (bei einer Temperatur von 21°C und Normaldruck).

Das erhaltene Amin besitzt eine Aminzahl von 19,20, die Ausbeute an Gesamtamin beträgt 97,3 Gew.-%, bezogen auf eingesetztes Ausgangsnitril. In diesem Gesamtamin beträgt der Anteil an sekundärem Amin 94,9 Mol-%, der Anteil an tertiärem Amin 3,9 Mol-% und der Anteil an primärem Amin 1,2 Mol-%. Die Iodzahl wird mit 9,1 ermittelt, das heißt, es wurden nur 82,6% der vorhandenen Doppelbindungen hydriert.

### Beispiel 2

Es wird entsprechend Beispiel 1 gearbeitet, wobei jedoch nach Passieren des Ammoniak-Wäschers nur ein Teilstrom des Kreisgases über den Trockenturm, gefüllt mit Silikagel, geleitet wird. Die vereinigten Teilströme haben noch einen Wassergehalt von 4,9 g/m³ bei 25°C und Normaldruck.

Das erhaltene Amin hat eine Aminzahl von 19,20, die Ausbeute an Gesamtamin beträgt 97,5 Gew.-%, bezogen auf eingesetztes Ausgangsnitril. Dieses Gesamtamin hat einen Anteil an sekundärem Amin von 96,3 Mol-%, an tertiärem Amin von 2,9 Mol-% und einen Anteil an primärem Amin von 0,8 Mol-%. Die Iodzahl ist 2,3, das heißt, es wurden 95,6% der vorhandenen Doppelbindungen hydriert.

### Beispiel 3

Es wird wie in Beispiel 1 gearbeitet, jedoch wird während der ersten drei Stunden der Reaktion nur soviel Wasser durch den Ammoniak-Waschturm geleitet, daß das Kreisgas nach Verlassen des Waschturms noch einen durchschnittlichen Ammoniakgehalt von 15 Vol.-% aufweist. Danach wird das Kreisgas wie in Beispiel 1 über einen mit Ätznatron gefüllten Trockenturm geleitet und hat danach einen Wassergehalt von 0,7 g/m³ bei 25°C und Normaldruck.

Das erhaltene Amin besitzt eine Aminzahl von 19,33, die Ausbeute an Gesamtamin ist 97,3 Gew.-%, bezogen auf eingesetztes Ausgangsnitril. Dieses Gesamtamin hat einen Anteil an sekundärem Amin von 94,0 Mol-%, an tertiärem Amin von 3,3 Mol-% und an primärem Amin von 2,7 Mol-%.

Die Iodzahl ist 0,9, das heißt, es wurden 98,3% der vorhandenen Doppelbindungen hydriert.

### Vergleichsbeispiel 3a)

Es wird wie in Beispiel 3 gearbeitet, jedoch wird das Kreisgas hier nicht über einen Trockenturm geleitet. Sein Wassergehalt beträgt wie in Beispiel 1 17,5 g/m³ (bei 21°C und Normaldruck).

Das erhaltene Amin hat eine Aminzahl von 19,57, die Gesamtamin-Ausbeute ist 98,5%, bezogen auf eingesetztes Ausgangsnitril. Der Anteil an sekundärem Amin beträgt 94,3 Mol-%, der Anteil an tertiärem Amin 3,0 Mol-% und der Anteil an primärem Amin 2,7 Mol-%.

Die Iodzahl ist jedoch noch 11,0, das heißt es wurden nur 79% der vorhandenen Doppelbindungen hydriert. Der Vergleich mit dem erfindungsgemäßen Beispiel 3 zeigt also, daß ein ammoniakhaltiges Kreisgas bei Gegenwart von Wassermengen, die über der Grenze des erfindungsgemäßen Verfahrens liegen, die Hydrierung der Doppelbindung noch wesentlich negativer beeinflußt als ein ammoniakfreies Kreisgas.

### Beispiel 4

In der oben beschriebenen Apparatur werden 200 g eines Talgfettnitrils (Stickstoffgehalt 5,52 Gew.-%, Iodzahl 45,4) und 5,0 g eines Nickelpulver-Katalysators (58% Nickel auf einem Träger aus $Al_2O_3/SiO_2$ etwa im Verhältnis 1 : 1) vorgelegt. Die Reaktionstemperatur beträgt 175°C, die Reaktionszeit 5 Stunden, die Kreisgasmenge 600 l/kg · h. Während der gesamten Reaktionszeit wird nur soviel Wasser durch den Ammoniak-Waschturm geleitet, daß das Kreisgas nach Verlassen des Waschturms noch einen durchschnittlichen Ammoniakgehalt von 40 Vol.-% aufweist (bei Abweichungen von ±3 Mol-%). Danach wird das Kreisgas über einen mit Ätznatron gefüllten Trockenturm geleitet und hat danach einen Wassergehalt von 0,6 g/m³ bei 25°C und Normaldruck.

Das erhaltene Amin besitzt eine Aminzahl von 19,58, die Gesamtamin-Ausbeute beträgt 98,8 Gew.-%, bezogen auf eingesetztes Ausgangsnitril. Das Gesamtamin besteht aus 2,1 Mol-% primärem, 93,1 Mol-% sekundärem und 4,8 Mol-% tertiärem Amin und hat eine Iodzahl von 2,0, das heißt, es sind 95,6% der vorhandenen Doppelbindungen hydriert worden.

### Vergleichsbeispiel 4a)

Es wird wie in Beispiel 4 gearbeitet, jedoch wird das Kreisgas nicht über einen Trockenturm geleitet. Sein Wassergehalt beträgt 15,9 g/m³ (bei 25°C und Normaldruck).

Dabei wird eine Gesamtamin-Ausbeute von 98,0 Gew.-% erhalten, die Aminzahl ist 19,47, was einem Anteil von 3,2 Mol-% an primärem, 91,5 an sekundärem und 5,3 Mol-% an tertiärem Amin entspricht. Die Iodzahl ist hier nach der angegebenen Reaktionszeit von 5 Stunden noch 21,0, das heißt es wurden nur 53,7% der vorhandenen Doppelbindungen hydriert.

### Beispiel 5

Im Reaktionskolben werden 200 g eines Talgfettnitrils (Stickstoffgehalt 5,4 Gew.-%, Jodzahl = 40,8) mit 5 Gew.-% Raney-Nickel (bezogen auf eingesetztes Nitril) vorgelegt, wobei vor der Hydrierung in der oben beschriebenen Weise weitgehend entwässert wird. Die Reaktionstemperatur beträgt 175°C. Der Waschturm der Apparatur wird mit Diethylenglykol berieselt, mit dem das bei der Reaktion gebildete Ammoniak aus dem Kreisgas herausgewaschen wird. Gleichzeitig wird durch das Diethylenglykol eventuell noch vorhandene Restfeuchtigkeit aus dem Kreisgas mitentfernt. Hinter dem Waschturm enthält das Kreisgas noch 0,8 g $H_2O/m^3$ bei 25°C und Normaldruck.

Nach 6 Stunden wird bei einer Gesamtaminausbeute von 99,4 Gew.-% ein Produkt der Aminzahl 19,48 erhalten, das aus 2,1 Mol-% primärem, 95,5 Mol-% sekundärem und 2,4 Mol-% tertiärem Amin besteht. Die Iodzahl ist 1,4, das heißt 96,5% der ursprünglich vorhandenen Doppelbindungen sind hydriert.

### Beispiel 6

Im Reaktionskolben werden 530 g eines Talgfettnitrils (Stickstoffgehalt 5,50 Gew.-%, Iodzahl = 49,4) mit 5 Gew.-% Raney-Nickel (bezogen auf Nitril) vorgelegt, wobei vor er Hydrierung in der oben beschriebenen Weise entwässert wird.

Die Reaktionstemperatur beträgt 175°C, die Kreisgasmenge beträgt 250 l/kg · h. Das Kreisgas passiert zur Adsorption des gebildeten Ammoniaks anstelle eines Waschturms einen Rührkolben, beschickt mit 1 Mol Talgfettsäure von 70°C pro Mol eingesetztes Talgfettnitril. Über die gesamte Reaktionszeit von 6 Stunden wird aus dem Kreisgas nach Passieren der Absorption insgesamt 3,5 l Abgas kontinuierlich ausgeschleust. Damit auf keinen Fall Säurespuren in den Reaktionskolben gelangen können, wird das Kreisgas nach Passieren des Rührkolbens durch eine Kältefalle (−30 bis −35°C) geleitet. Das Kreisgas enthält 0,3 g/m³ Wasser bei 25°C und Normaldruck. Das Endprodukt hat die Aminzahl 19,07 und besteht aus 1,2 Mol-% primärem, 96,0 Mol-% sekundärem und 2,8 Mol-% tertiärem Amin. Die Gesamtaminausbeute ist 96,1 Gew.-%, bezogen auf eingesetztes

Nitril. Die Iodzahl beträgt 1,5, das heißt 97% der Doppelbindungen sind hydriert.

### Beispiel 7

Es wird wie im Beispiel 1 gearbeitet, das Kreisgas läuft während der gesamten Reaktionszeit in seiner ganzen Menge über einen mit Wasser berieselten Ammoniak-Waschturm und anschließend über einen Trockenturm, gefüllt mit Ätznatron. Das Kreisgas hat nach der Trocknung einen Wassergehalt von 0,7 g/m³ bei 25°C und Normaldruck.

Es werden 200 g Ölsäurenitril (Stickstoffgehalt 5,20 Gew.-%, Iodzahl = 96,3) eingesetzt. Als Katalysator dient 5 Gew.-% Raney-Nickel (bezogen auf Nitril); Reaktionstemperatur 175°C. Bei einer Gesamtaminausbeute von 99,2 Gew.-% wird ein Produkt der Aminzahl 18,55 erhalten, das aus 1,3 Mol-% primärem, 95,4 Mol-% sekundärem und 3,3 Mol-% tertiärem Amin besteht. Die Iodzahl ist 1,7, das heißt 98,2% der im Ausgangsprodukt vorhanden gewesenen Doppelbindungen sind hydriert.

### Vergleichsbeispiel 7a

Es wird wie in Beispiel 7 gearbeitet, jedoch das Kreisgas anschließend an den mit Wasser berieselten Waschturm nicht getrocknet. Nach dem Passieren des Waschturms hat das Kreisgas einen Wassergehalt von 17,5 g/m³ bei einer Temperatur von 21°C und Normaldruck.

Das erhaltene Produkt hat eine Aminzahl von 18,37, die Ausbeute an Gesamtamin beträgt 98,7 Gew.-%, bezogen auf das eingesetzte Nitril. Dieses Gesamtamin setzt sich zusammen aus 1,1 Mol-% primärem, 94,8 Mol-% sekundärem und 4,1 Mol-% tertiärem Amin. Die Iodzahl wird zu 16,3 ermittelt, das heißt, nur 83,1% der Doppelbindungen im Ausgangsprodukt sind hydriert.

### Beispiel 8

In der gleichen Apparatur wie in Beispiel 7 werden 200 g Sojanitril (Stickstoffgehalt 5,21 Gew.-%, Iodzahl 124,7) mit 5 Gew.-% Raney-Nikkel (bezogen auf Nitril) eingesetzt. Das Sojanitril setzt sich zusammen aus 22 Gew.-% gesättigten Nitrilen einer Kettenlänge von 14, 16 und 18 C-Atomen. 28 Gew.-% $C_{18}$-Nitril mit 1 Doppelbindung in der Kohlenstoffkette (Ölsäurenitril), 48 Gew.-% $C_{18}$-Nitril mit 2 Doppelbindungen (Linolsäurenitril) und 2 Gew.-% $C_{18}$-Nitril mit 3 Doppelbindungen (Linolensäurenitril). Es wird bei 175°C hydriert. Die gesamte Kreisgasmenge von 500 l/kg · h wird zur Entfernung des bei der Reaktion gebildeten Ammoniaks über einen mit Wasser berieselten Waschturm geführt und danach über einen Trockenturm mit Ätznatron geleitet. Das Kreisgas hat hinter der Trocknung einen Wassergehalt von 0,6 g/m³ bei 25°C und Normaldruck. Nach 6 Stunden wird bei einer Gesamtaminausbeute von 99,1 Gew.-% ein Produkt der Aminzahl 18,61 erhalten, das aus 1,2 Mol-% primärem, 96,0 Mol-% sekundärem und 2,8 Mol-% tertiärem Amin besteht. Die Iodzahl ist 1,1, es sind also 99,1% der im Ausgangsprodukt vorhanden gewesenen Doppelbindungen hydriert.

### Vergleichsbeispiel 8a)

Es wird wie in Beispiel 8 gearbeitet, jedoch wird das Kreisgas nach der Ammoniakwäsche nicht getrocknet. Sein Wassergehalt beträgt wieder 17,5 g/m³ bei 21°C und Normaldruck. Bei einer Gesamtaminausbeute von 98,4 Gew.-%, bezogen auf eingesetztes Sojanitril, resultiert ein Produkt der Aminzahl 18,42, das aus 1,0 Mol-% primärem, 95,7 Mol-% sekundärem und 3,3 Mol-% tertiärem Amin besteht. Die Iodzahl wird zu 19,1 gefunden, das heißt, nur 84,7% der ursprünglich vorhandenen Doppelbindungen sind hydriert.

Die angegebenen Zahlen wurden durch Titration mit n/10 Perchlorsäure in Eisessig ermittelt. Sie ist definiert als Verbrauch an n/10 Perchlorsäure in ml pro g Substanz.

Die Iodzahl wurde bestimmt durch Titration mit Bromid-Bromat-Lösung in Eisessig nach der »dead-stop«-Methode und Umrechnung des erhaltenen Verbrauchs auf Iodzahlen (Verbrauch g Iod/100 g Substanz). Zur Vermeidung von Nebenreaktionen wurde das Amin vorher mit Essigsäureanhydrid acetyliert.

### Patentansprüche

1. Verfahren zur Herstellung gesättigter sekundärer Alkylamine mit 12 bis 22 Kohlenstoff-Atomen in jeder Alkylkette, wobei diese gleich oder verschieden sein können, durch Hydrierung von ungesättigten Fettsäurenitrilen der entsprechenden Kettenlängen mit 1 bis 3 olefinischen Doppelbindungen im Molekül oder deren Gemischen der genannten Kettenlängen in Gegenwart von Nickel- oder Kobalt-Katalysatoren, wobei der Wasserstoff im wesentlichen im Kreis geführt und Ammoniak aus dem Kreisgas entfernt wird, dadurch gekennzeichnet, daß unter praktisch drucklosen Bedingungen während der gesamten Reaktionsdauer im Kreisgas vor Rückführung in die Reaktionszone ein Wassergehalt von höchstens 5 g/m³ bei 25°C und Normaldruck eingestellt und/oder aufrechterhalten wird.

2. Verfahren nach Anspruch 1, dadurch gekennzeichnet, daß nach Entfernung des Ammoniaks durch Waschen mit Wasser das Kreisgas über ein Trockenmittel geführt wird, welches den Wassergehalt des Kreisgases auf einen Wert von höchstens 5 g/m³ bei 25°C und Normaldruck herabsetzt.

3. Verfahren nach Anspruch 1, dadurch gekennzeichnet, daß die Entfernung des Ammoniaks durch im wesentlichen wasserfreie Absorptionsmittel für Ammoniak erfolgt.

4. Verfahren nach Anspruch 1 bis 3, dadurch gekennzeichnet, daß während der Reaktion 1 bis 20 Vol.-% des umlaufenden Kreisgases aus dem Kreislauf ausgeschleust werden.

## Claims

1. A process for the manufacture of saturated secondary alkylamines having 12 to 22 carbon atoms in each alkyl chain which can be identical or different, by hydrogenation of unsaturated fatty acid nitriles having corresponding chain length and 1 to 3 olefinic double bonds in the molecule, or mixtures thereof with up to 70% by weight of saturated fatty acid nitriles of the said chain length, in the presence of nickel or cobalt catalysts, the hydrogen substantially being circulated and ammonia being removed from the circulating gas which is characterised by setting and/or maintaining a water content of at most 5 g/m³ at 25° C and under normal pressure, in the circulating gas under virtually unpressurized conditions before recycle to the reaction zone for the entire duration of the reaction.

2. A process as claimed in claim 1 which is characterised in that after removal of the ammonia by scrubbing with water, the circulating gas is passed over a drying agent which lowers the water content of the circulating gas to a value of at most 5 g/m³ at 25°C and under normal pressure.

3. A process as claimed in claim 1 which ist characterised in that the removal of ammonia is effected by means of substantially anhydrous absorbents for ammonia.

4. A process as claimed in any of claims 1 to 3 which is characterised in that 1 to 20% by volume of the circulating recycle gas are withdrawn from the circulation during the reaction.

## Revendications

1. Procédé de préparation d'alkylamines secondaires saturées contenant de 12 à 22 atomes de carbone dans chacune des chaînes alkyles, lesquelles peuvent être identiques ou différentes, par hydrogénation de nitriles d'acides gras insaturés aux longueurs de chaînes correspondantes et contenant de 1 à 3 doubles liaisons oléfiniques dans la molécule ou de leurs mélanges avec une proportion allant jusqu'à 70% en poids de nitriles d'acides gras saturés aux longueurs de chaînes mentionnées, en présence de catalyseurs au nickel ou au cobalt, l'hydrogène étant essentiellement recyclé et l'ammoniac étant éliminé des gaz de recyclage, procédé caractérisé en ce que, à pression pratiquement normale et pendant toute la durée de réaction, on règle et/ou on maintient dans les gaz de recyclage, avant retour dans la zone de réaction, une teneur en humidité qui est au maximum de 5 g/m³ à 25° C et pression normale.

2. Procédé selon la revendication 1, caractérisé en ce que, après avoir éliminé l'ammoniac par lavage à l'eau, on envoie les gaz de recyclage sur un agent déshydratant qui abaisse la teneur en humidité des gaz de recyclage à un niveau de 5 g/m³ au maximum à 25°C et pression normale.

3. Procédé selon la revendication 1, caractérisé en ce qu'on élimine l'ammoniac au moyen d'agents adsorbants de l'ammoniac essentiellement anhydres.

4. Procédé selon les revendications 1 à 3, caractérisé en ce que, durant la réaction, on évacue du circuit de 1 à 20% en volume des gaz de recyclage en circulation.